# EUROPÄISCHE PATENTSCHRIFT

(11) **EP 1 344 494 B1**
(45) Veröffentlichungstag und Bekanntmachung des Hinweises auf die Patenterteilung: **16.03.2005**
(21) Anmeldenummer: 03004768.2
(22) Anmeldetag: 05.03.2003
(51) Int. Cl.: A61B 17/17

(54) **Zielgerät für Verriegelungsnägel**
Aiming apparatus for locking nail
Appareil de visée pour clou verrouillable

(30) Priorität: 15.03.2002 DE 20204126 U
(43) Veröffentlichungstag der Anmeldung: 17.09.2003
(73) Patentinhaber: Stryker Trauma GmbH, 24232 Schönkirchen (DE)
(72) Erfinder: Pusnik, Peter, 24103 Kiel (DE); Bigdeli, Sabine, 24242 Felde (DE)
(74) Vertreter: Patentanwälte Hauck, Graalfs, Wehnert, Döring, Siemons, Schildberg

(56) Entgegenhaltungen:
- WO-A-03/041595
- US-A- 5 295 991
- US-A- 5 334 192
- US-A- 5 346 496
- PATENT ABSTRACTS OF JAPAN vol. 2002, no. 02, 2. April 2002 (2002-04-02) & JP 2001 286480 A (HOMUZU GIKEN:KK), 16. Oktober 2001 (2001-10-16)

## Beschreibung

Die Erfindung bezieht sich auf ein Zielgerät für Verriegelungsnägel nach dem Oberbegriff des Anspruchs 1.

Verriegelungsnägel sind bekanntlich Knochennägel, die mit Querdurchbohrungen versehen sind, durch welche Knochenschrauben hindurchgeführt werden. Für die Herstellung von Bohrungen im Knochen zum Einführen der Knochenschrauben ist bekanntlich erforderlich, eine Zielvorrichtung zu verwenden. Eine typische Zielvorrichtung oder ein Zielgerät wird fest mit dem Ende des Knochennagels verbunden, auf den die Eintreibkraft aufgebracht wird. Beim Einführen in den Femur ist dies normalerweise das proximale Ende des Nagels. Der Befestigungsabschnitt des Zielgeräts ist über einen zumeist bügelförmigen Abschnitt mit einem Zielarm verbunden. Im Zielarm sind Querbohrungen vorgesehen, die zu den Bohrungen im Verriegelungsnagel ausgerichtet sind, wenn dieser mit dem Befestigungsabschnitt in der beschriebenen Weise verbunden ist. Da, wie erwähnt, der Verriegelungsnagel mehrere Querbohrungen hat, unter anderem auch eine Schrägdurchbohrung für eine Schenkelhalsschraube, weisen die einzelnen Querbohrungen einen unterschiedlichen Winkel auf. Die Bohrungen im Zielarm sind mithin analog ausgebildet.

Bei der Herstellung einer Bohrung im Knochen wird naturgemäß nur die zugeordnete Bohrung im Zielarm benötigt. Durch diese wird dann eine Zielund Bohrhülse geschoben, welche bis zum Knochen vorgeschoben wird, über die dann der erste Einstich in den Knochen und das anschließende Bohren erfolgt. Damit der Operateur nicht versehentlich eine falsche Bohrung wählt, ist ferner bekannt, über den Zielarm eine Hülse zu schieben. Die Hülse enthält eine Querbohrung oder ein Paar von Querlöchern, welche zu einer Querbohrung im Zielarm ausgerichtet ist, wenn die Hülse auf den Zielarm geschoben ist. Sind z.B. vier verschiedene Durchbohrungen im Zielarm vorgesehen, sind vier Hülsen pro Zielgerät vorzuhalten. Da bei einem Verriegelungsnagel eine Reihe von Zielhülsen benötigt werden, ist daher jeweils die zugeordnete Hülse auszuwählen und auf den Zielarm aufzuschieben. Dies bedeutet einen nicht unbeträchtlichen Aufwand.

Dokument US-A-5,334,192 offenbart ein Zielgerät nach dem Oberbegriff des Anspruchs 1.

Der Erfindung liegt die Aufgabe zugrunde, ein Zielgerät zu schaffen, bei dem sowohl der Aufwand für die Herstellung als auch der Operationsaufwand verringert ist.

Diese Aufgabe wird durch die Merkmale des Anspruchs 1 gelöst.

Bei der Erfindung wird auf den Zielarm eine Verdrehhülse drehbar, jedoch im wesentlichen axial unverschiebbar gelagert, die eine Reihe von in Umfangsrichtung und axial beabstandete Queröffnungen aufweist, wobei in vorgegebenen Drehstellungen der Verdrehhülse jeweils eine Öffnung mit einer Bohrung des Zielarms ausgerichtet ist. Bei dem erfindungsgemäßen Zielgerät ist mithin nur eine Hülse auf dem Zielarm anzuordnen, die ständig auf dem Zielarm verbleibt. Je nach Drehstellung kann jeweils ein Paar Queröffnungen zu einer Querbohrung im Zielarm ausgerichtet werden. Am Zielarm bzw. an der Zielhülse können entsprechende Markierungen vorgesehen werden, durch welche der Operateur leicht erkennen kann, bei welcher Drehstellung der Verdrehhülse eine Querbohrung des Zielarms zum Einsatz gelangt. Da die Verdrehhülse ohne besondere Vorkehrungen unwillkürlich eine Verdrehung erfahren kann, ist es ferner vorteilhaft, wenn bei dem erfindungsgemäßen Zielgerät Rastmittel vorgesehen sind, welche die Verriegelungshülse auf dem Zielarm in einer eingenommenen Drehstellung rastend verriegelt.

In einer eingenommenen Drehstellung wird, wie beschrieben, eine Ziel- und Bohrhülse durch die Öffnungen der Verdrehhülse und durch die Querbohrung des Zielarms hindurchgeführt bis zur Anlage an den Knochen. Falls keine Vorkehrungen getroffen werden, kann eine ungewollte Verschiebung der Ziel- und Bohrhülse erfolgen. Um eine solche zu vermeiden, sieht eine Ausgestaltung der Erfindung vor, daß mit dem Zielarm eine Spanneinrichtung verbunden ist, mit der eine geringfügige axiale Verschiebung der Verdrehhülse auf dem Zielarm erzeugt wird zur Klemmung einer Ziel- und Bohrhülse, welche von einer Bohrung des Zielarms und von den Öffnungen der Verdrehhülse aufgenommen ist. Für die Verklemmung einer Ziel- und Bohrhülse reicht nur eine minimale axiale Verschiebung der Verdrehhülse aus. Diese axiale Verschiebung ist im Rahmen der Toleranz möglich, welche durch die an sich axiale Festlegung der Verschiebehülse besteht.

Damit die Hülse annähernd axial unverschiebbar auf dem Zielarm gelagert werden kann, sieht eine Ausgestaltung der Erfindung vor, daß die Hülse am hinteren Ende einen inneren radialen Vorsprung aufweist, der mit einer Ringnut des Zielarms zusammenwirkt. Dieser Vorsprung kann auch dazu verwendet werden, mit Rastausnehmungen des Zielarms zusammenzuwirken, um in den vorgegebenen Drehstellungen der Verdrehhülse diese auf dem Zielarm zu verrasten.

Zur geringfügigen axialen Verschiebung der Spannhülse zwechs Einklemmens der Ziel- und Bohrhülse sind verschiedene konstruktive Möglichkeiten denkbar. Eine sieht erfindungsgemäß vor, daß die Spanneinrichtung einen Drehknopf aufweist, der am freien Ende der Verdrehhülse liegend mittels eines mittigen Zapfens in einer Ausnehmung am freien Ende des Zielarms verdrehbar, jedoch axial unverschiebbar angebracht ist. Der Druckknopf und die Verdrehhülse wirken über eine Führungskurve und einen Kurvenfolger so zusammen, daß bei einer Drehung des Drehknopfs die Verdrehhülse in axialer Richtung vorgespannt wird. Zu diesem Zweck kann der Drehknopf topfförmig ausgebildet sein und an der Innenseite am Boden mittig einen axialen Ansatz aufweisen, an dessen Ende ein Zapfen angeordnet ist, der mit einer Ausnehmung des Zielarms zusammenwirkt. Am Umfang des Ansatzes ist eine Ringnut angeordnet, die mit mindestens einem radialen Vorsprung der Verdrehhülse zusammenwirkt, wobei die Ringnut über eine achsparallele Nut mit dem freien Ende des Ansatzes verbunden ist zum Einführen des Vorsprungs in die Ringnut. Die Ringnut hat eine gewisse Neigung, so daß bei einer Verdrehung der Ringnut die Spannhülse in axialer Richtung vorgespannt wird.

Eine weitere Ausgestaltung der Erfindung sieht vor, daß zwischen dem Druckknopf und dem freien Ende der Verdrehhülse eine Feder angeordnet ist, die die Teile gegeneinander vorspannt.

Ein Ausführungsbeispiel der Erfindung wird nachfolgend anhand von Zeichnungen näher erläutert.
- Fig. 1: zeigt perspektivisch ein Zielgerät nach der Erfindung.
- Fig. 2: zeigt die Seitenansicht des Zielgeräts nach Fig. 1 ohne Verdrehhülse.
- Fig. 3: zeigt einen Schnitt durch die Darstellung nach Fig. 2 entlang der Linie 3-3.
- Fig. 4: zeigt perspektivisch die Verdrehhülse des Zielgeräts nach Fig.1 einschließlich eines Verdrehknopfes.
- Fig. 5: zeigt die Endansicht der Verdrehhülse nach Fig. 4 in Richtung Pfeil 5.
- Fig. 6: zeigt einen Schnitt durch die Verdrehhülse nach Fig. 5 entlang der Linie 6-6.
- Fig. 7: zeigt einen Schnitt durch die Verdrehhülse nach Fig. 5 entlang der Linie 7-7, wobei nur ein Teil der Länge der Hülse dargestellt ist.
- Fig. 8: zeigt in Seitenansicht den Verdrehknopf nach Fig. 4.
- Fig. 9: zeigt einen Schnitt durch den Verdrehknopf nach Fig. 8.
- Fig. 10 und 9.: zeigt perspektivisch den Ansatz des Drehknopfes nach den Fign. 8

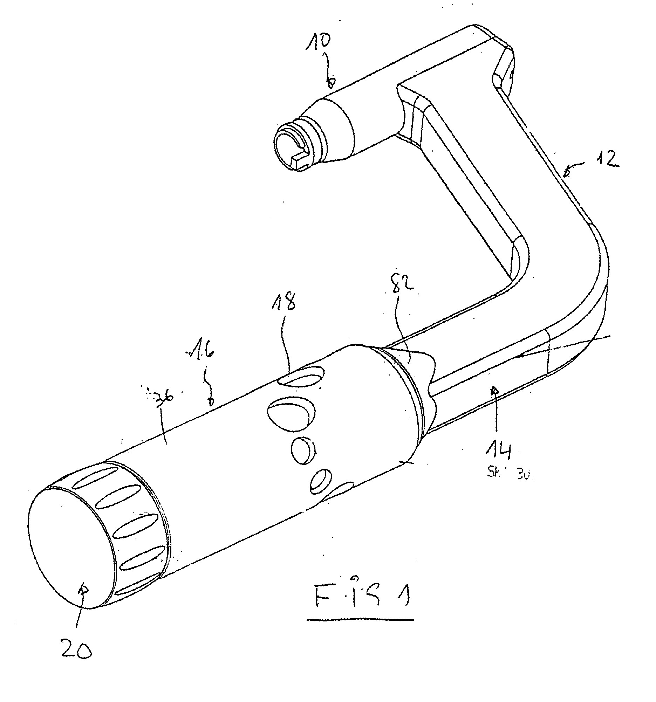

Das in Fig. 1 dargestellt Zielgerät weist einen Befestigungsabschnitt 10 auf, der in geeigneter Weise mit einem Ende eines nicht gezeigten Verriegelungsnagels verbunden werden soll. Diese Befestigung soll nicht erörtert werden. Sie gehört zum Stand der Technik.

Mit dem Befestigungsabschnitt 10 ist über einen bügelförmigen Verbindungsabschnitt 12 ein Zielarm 14 fest verbunden. Auf dem Zielarm 14 ist eine Verdrehhülse 16 drehbar gelagert. Die Verdrehhülse 16 weist über ihren Umfang mehrere Queröffnungen 18 auf, die axial unterschiedlich angeordnet und in unterschiedlichem Winkel ausgebildet sind. Auf den Zweck dieser Öffnung wird weiter unten noch eingegangen.

Am freien Ende der Zielhülse 16 ist ein Drehknopf 20 angeordnet.

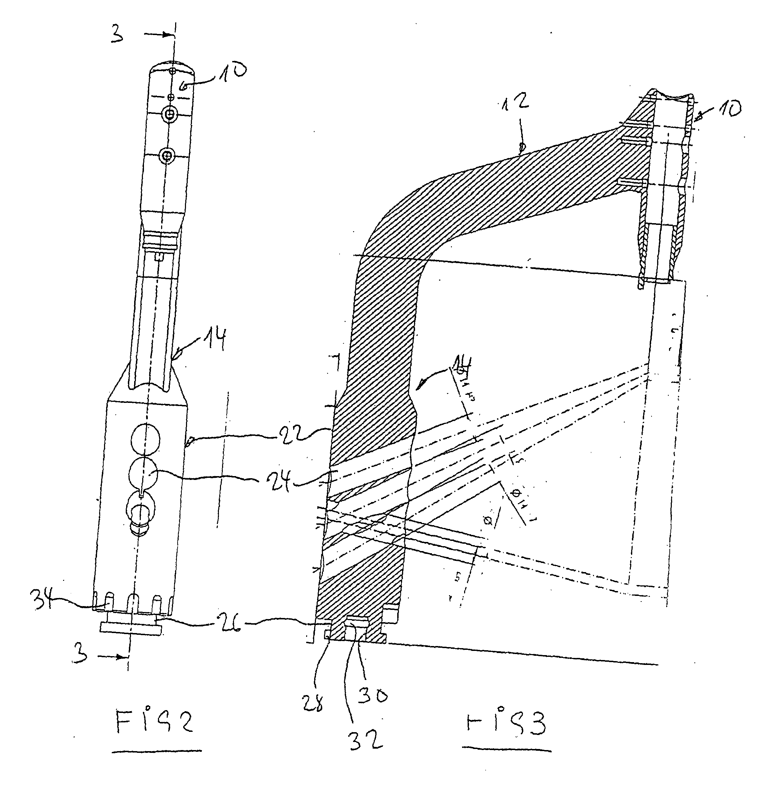

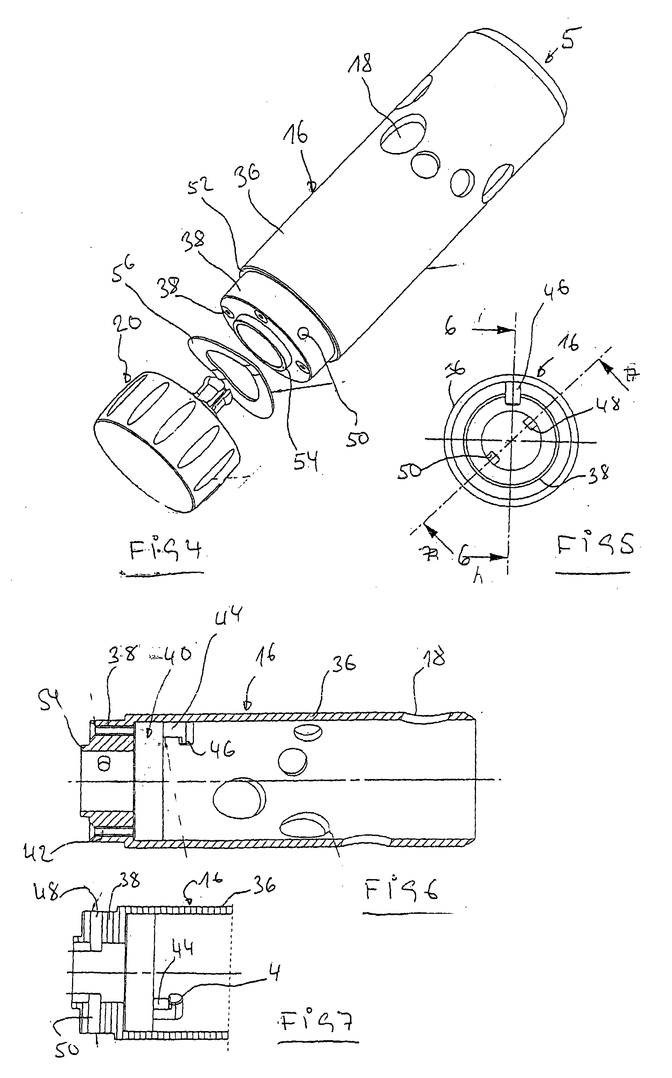

Befestigungsabschnitt 10, Verbindungsabschnitt 12 und Zielarm 14 gehen deutlicher aus den Fign. 2 und 3 hervor. Wie zu erkennen, hat der Zielarm 14 im unteren Bereich einen annähernd zylindrischen Abschnitt 22, der mit einer Reihe von Querbohrungen 24 versehen ist. Es sind insgesamt 4 Querbohrungen vorgesehen, die sämtlich in einem unterschiedlichen Winkel angeordnet sind, von denen eine Bohrung einer Doppelbohrung entspricht. Die Achsen der Querbohrungen 24 sind zu Achsen von Löchern eines nicht gezeigten Verriegelungsnagels ausgerichtet, wenn dieser mit dem Befestigungsabschnitt 10 fest verbunden ist.

Nahe dem freien Ende weist der Zielarm 14 eine Ringnut 26 auf, welche durch eine achsparallele Nut 28 mit dem freien Ende des Zielarms 14 verbunden ist Stirnseitig ist vom freien Ende her eine Ausnehmung 30 geformt mit einer inneren Ringnut 32, die einen Hinterschnitt bildet. Aus Fig. 2 ist zu entnehmen, daß angrenzend in die Wandfläche der Ringnut 26 in Umfangsrichtung-beabstandet Rastausnehmungen 34 geformt sind, deren Funktion noch erläutert wird.

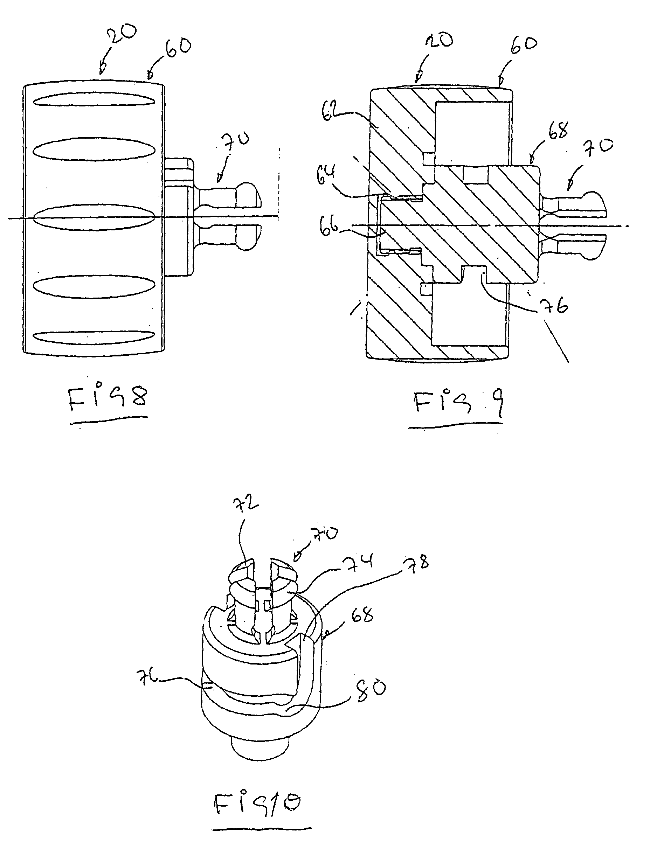

Aus den Fign. 4 bis 7 geht der Aufbau der Verdrehhülse 16 hervor. Der Innendurchmesser des Abschnitts 36, der sich weitgehend über die ganze Länge der Verdrehhülse 16 erstreckt, ist geringfügig größer als der des zylindrischen Abschnitts 22 des Zielarms 14. Links davon erstreckt sich ein Abschnitt 38 mit einem etwas geringeren Innendurchmesser. Gegen die dadurch gebildete Stufe ist ein Blockierring 40 gelegt, der mit Hilfe von Befestigungsschrauben befestigt ist, welche über achsparallele Bohrungen 42 eingeschraubt werden. Der Blockierring 40 weist an einem achsparallelen Ansatz 44 einen radial nach innen weisenden Vorsprung 46 auf. In dem Abschnitt 38 sind diametral gegenüberliegend zwei radiale Stifte 48, 50 im Preßsitz angeordnet, die etwas in das Innere des Abschnitts 38 hinein stehen.

Ferner erkennt man, daß der Außendurchmesser des Abschnitts 38 etwas geringer ist als der Außendurchmesser des Abschnitts 36, so daß eine entsprechende Schulter 52 gebildet ist. Schließlich weist der Abschnitt 38 am freien Ende einen axialen Bund 54 auf, der zur Aufnahme einer Scheibenfeder 56 dient, welche auf den Bund plaziert wird in Anlage an die Stirnseite des Abschnitts 38 der Verdrehhülse 16.

Der Aufbau des Drehknopfes 20 geht aus den Fign. 8 bis 10 hervor. Er weist ein topfförmiges Knopfteil 60 auf, dessen Boden 62 eine Ausnehmung 64 aufweist. In die Ausnehmung ist ein axialer Vorsprung 66 eines axialen Ansatzes 68 eingesetzt und dort entweder durch Klebung oder eine Gewindeverbindung befestigt. Der Ansatz weist am rechten Ende einen Zapfen 70 auf, der als Clip geformt ist mit vier im gleichen Umfangsabstand und im Abstand angeordneten Armen 72, die eine radiale Erweiterung 74 am äußeren Ende aufweisen.

Der Ansatz 68 weist ferner diametral gegenüberliegend zwei Ringnutabschnitte 76 auf, die über einen achsparallelen Nutabschnitt 78 mit dem oberen Enderdes Ansatzes 68 verbunden sind. Am inneren Ende des achsparallelen Nutabschnitts 78 ist eine Vertiefung 80 vorgesehen. Der Ringnutabschnitt 76 ist von der Vertiefung 80 an nach links mit einem kleinen Gefälle versehen. Es versteht sich, daß dies für den Ringnutabschnitt auf der gegenüberliegenden Seite gleichermaßen gilt.

Bei der Montage wird die Verdrehhülse 78 auf den zylindrischen Abschnitt 22 des Zielarms 14 aufgeschoben, wobei der radiale Vorsprung 46 über die achsparallele Nut 78 in die Ringnut 76 eingreifen kann, wenn die Hülse etwas verdreht wird. Die Rastausnehmungen 34 ermöglichen eine Festlegung der Verdrehstellung der Verdrehhülse 16 in vorgegebenen Drehpositionen dergestalt, daß jeweils eine Öffnung 18 der Verdrehhülse 16 zu einer Bohrung 24 des Zielarms 14 ausgerichtet ist.

Der Zapfen 70 des Ansatzes 68 wird durch die Scheibenfeder 56 und den Bund 54 hindurch in die Ausnehmung 30 eingesteckt, wobei die Erweiterungen 74 des Zapfens 70 in die Ringnut 32 einrasten. Dadurch ist der Verdrehknopf axial gesichert, kann jedoch verdreht werden. Beim Einführten des Zapfens 70 in der beschriebenen Art greift der Ansatz 68, der in den Fign. 8 bis 10 vergrößert dargestellt ist, in das Innere des Abschnitts 38 der Hülse 16. Hierbei ist der Drehknopf 20 in seiner Drehlage so anzuordnen, daß die achsparallelen Nuten 78 zu den Stiften 48, 50 ausgerichtet sind. Sobald die Vertiefung 80 erreicht ist, wird der Drehknopf 20 etwas in Uhrzeigerrichtung verdreht, bis die Stifte 48, 50 in den Ringnutabschnitt 76 einlaufen. Eine weitere Verdrehung des Drehknopfs 70 führt dazu, daß die Verdrehhülse 76 in Richtung Drehknopf gezogen wird. Befindet sich in einer Querbohrung 74 und einer entsprechenden Öffnung 18 eine hier nicht dargestellte Ziel- und Bohrhülse, wird bei der beschriebenen Verschiebung der Verdrehhülse diese festgeklemmt.

Bei der beschriebenen Anordnung befindet sich ein topfförmiger Drehkopfteil auf dem Abschnitt 38 der Hülse 16 wie sich aus Fig. 1 ergibt. Daraus ist auch zu erkennen, daß der Außenumfang des gerändelten Drehknopfs 20 annähernd den gleichen Durchmesser hat wie der Abschnitt 36 der Verdrehhülse 16. Zwischen der Verdrehhülse 16 und dem angrenzenden Abschnitt des Zielarms 14, welch letzterer im Querschnitt rechteckig geformt ist, ist ein entsprechender weicher Übergang 82 geformt.

## Patentansprüche

1. Zielgerät für Verriegelungsnägel mit einem ersten Abschnitt (10), der fest mit dem Ende eines Verriegelungsnagels verbindbar ist, einem Zielarm (14), der über einen bügelartigen Verbindungsabschnitt (12) mit dem ersten Abschnitt verbunden ist und mehrere Bohrungen (24) aufweist zur Aufnahme einer Ziel- und Bohrhülse, wobei die Bohrungen in unterschiedlichen Winkeln angeordnet sind, **dadurch gekennzeichnet, daß** auf dem Zielarm (14) eine Verdrehhülse (16) drehbar, jedoch im wesentlichen axial unverschiebbar gelagert ist, die eine Reihe von in Umfangsrichtung und axial beabstandeten Queröffnungen (18) aufweist, wobei in vorgegebenen Drehstellungen der Verdrehhülse (16) jeweils eine Öffnung (18) mit einer Bohrung des Zielarms (14) ausgerichtet ist und Rastmittel vorgesehen sind, welche die Verdrehhülse (16) auf dem Zielarm (14) in den einzelnen Drehstellungen rastend verriegeln.

2. Zielgerät nach Anspruch 1, **dadurch gekennzeichnet, daß** mit dem Zielarm (14) eine Spannvorrichtung verbunden ist, mit der eine geringfügige axiale Verschiebung der Verdrehhülse (16 ) auf dem Zielarm (14) erzeugt wird zur Klemmung einer Ziel- und Bohrhülse, welche von einer Bohrung des Zielarms (14) und einer Öffnung (18) der Verdrehhülse (16) aufgenommen ist.

3. Zielgerät nach einem der Ansprüche 1 bis 2, **dadurch gekennzeichnet , daß** die Hülse (16) am hinteren Ende einen inneren Vorsprung (46) aufweist, der mit einer Ringnut (26) des Zielarmes (14) zusammenwirkt.

4. Zielgerät nach Anspruch 3, **dadurch gekennzeichnet, daß** der Vorsprung (24) auch mit Rastausnehmungen (34) des Zielarms (14), die der Ringnut (26) zugekehrt sind, zusammenwirkt.

5. Zielgerät nach einem der Ansprüche 1 bis 4, **dadurch gekennzeichnet, daß** die Spanneinrichtung einen Drehknopf (20) aufweist, der am freien Ende der Verdrehhülse (16) liegend mittels eines mittigen Zapfens (70) in einer Ausnehmung (30) des Zielarms verdrehbar angebracht ist und der Drehknopf (20) und die Verdrehhülse (16) über eine Führungskurve und einen Kurvenfolger so zusammenwirken, daß bei einer Drehung des Drehknopfes (20) die Verdrehhülse (16) in axialer Richtung vorgespannt wird.

6. Zielgerät nach Anspruch 5, **dadurch gekennzeichnet, daß** der Drehknopf (20) topfförmig ist und an der Innenseite am Boden mittig einen axialen Ansatz (68) aufweist, an dessen oberen Ende der Zapfen (70) angeordnet ist und der am Umfang einen Ringnutabschnitt (76) aufweist, der mit einem radialen Vorsprung (48, 50) der Verdrehhülse (16) zusammenwirkt, wobei der Ringnutabschnitt (76) über eine achsparallele Nut (78) mit dem freien Ende des Ansatzes (68) verbunden ist zwecks Einführung des Vorsprungs (48, 50) in den Ringnutabschnitt (76).

7. Zielgerät nach Anspruch 6, **dadurch gekennzeichnet, daß** der Ansatz (68) als getrenntes Teil in dem topfförmigen Drehknopf (20) befestigt ist.

8. Zielgerät nach einem der Ansprüche 5 bis 7, **dadurch gekennzeichnet, daß** zwischen dem Drehknopf (20) und dem freien Ende der Verdrehhülse (16) eine Feder angeordnet ist, die die Teile gegeneinander vorspannt.

9. Zielgerät nach Anspruch 8, **dadurch gekennzeichnet, daß** eine Scheibenfeder (56) vorgesehen ist.

## Claims

1. An aim-taking apparatus for locking nails, comprising a first portion (10) which is adapted to be fixedly connected to the end of a locking nail, an aim-taking arm (14) which is connected to the first portion via a shackle-shaped connecting portion (12) and has a plurality of bores to receive an aim-taking and drilling sleeve wherein the bores (24) are disposed at different angles, **characterized in that** the aim-taking arm (14) has supported thereon a rotatable sleeve (16) which is ro-tatable, but substantially is axially displaceable and has a series of cross-bores (18) which are circumferentially and axially spaced wherein, in the predetermined rotational positions of the rotatable sleeve (16), one aperture each (18) is aligned with a bore of the aim-taking arm (14) and detent means are provided which catchingly lock the rotatable sleeve (16) on the aim-taking arm (14) in the individual rotational positions.

2. The aim-taking apparatus as claimed in claim 1, **characterized in that** the aim-taking arm (14) has connected thereto a tightening device which produces a slight axial displacement of the rotatable sleeve (16) on the aim-taking arm (14) to clip an aim-taking and drilling sleeve which is received by a bore of the aim-taking arm (14) and an opening (18) of the rotatable sleeve (16).

3. The aim-taking apparatus as claimed in any one of claims 1 to 2, **characterized in that** the sleeve (16), at the rear end, has an internal projection (46) which cooperates with an annular groove (26) of the aim-taking arm (14).

4. The aim-taking apparatus as claimed in claim 3, **characterized in that** the projection (24) also cooperates with detent recesses (34) of the aim-taking arm (14) which face the annular groove (26).

5. The aim-taking apparatus as claimed in any one of claims 1 to 4, **characterized in that** the tightening device has a rotary knob (20) which, lyeing at the free end of the rotatable sleeve (16), is rotatably mounted by means of a central trunnion (70) in a recess (30) of the aim-taking arm, and the rotary knob (20) and the rotatable sleeve (16) cooperate via a guide cam and a cam follower so that, upon rotation of the rotary knob (20), the rotatable sleeve (16) is biased in an axial direction.

6. The aim-taking apparatus as claimed in claim 5, **characterized in that** the rotary knob (20) is pot-shaped. and, centrally at the inside at the bottom, has an axial lug (68) at the upper end of which the trunnion (70) is disposed, and which circumferentially has an annular groove portion (76) which cooperates with a radial protrusion (48, 50) of the rotatable sleeve (16), the annular groove portion (76) being connected to the free end of the lug (68) via an axially parallel groove (78) for the purpose of introducing the protrusion (48, 50) into the annular groove portion (76).

7. The aim-taking apparatus as claimed in claim 6, **characterized in that** the lug (68) is mounted as a separate part in the pot-shaped rotary knob (20).

8. The aim-taking apparatus as claimed in any one of claims 5 to 7, **characterized in that** a spring which biases the component against each other is disposed between the rotary knob (20) and the free end of the rotatable sleeve (16).

9. The aim-taking apparatus as claimed in claim 8, **characterized in that** a curved washer (56) is provided.

## Revendications

1. Appareil de visée pour clou de verrouillage comportant un premier élément (10), qui peut être raccordé de façon fixe à l'extrémité d'un clou de verrouillage, un bras de visée (14), qui est relié, par un élément de liaison (12) en forme d'étrier, au premier élément (10) et présente plusieurs alésages (24) pour recevoir une douille de visée et de forage, les alésages étant disposés sous des angles différents,
**caractérisé en ce que**, sur le bras de visée (14), une douille rotative (16) est montée de manière à pouvoir pivoter, mais essentiellement sans pouvoir coulisser axialement, qui présente une série d'ouvertures transversales (18) à distance suivant la direction périphérique et axialement, dans des positions de rotation prédéterminées de la douille rotative (16), une ouverture (18) étant chaque fois alignée avec un alésage du bras de visée (14) et des moyens de blocages étant prévus, qui verrouillent en la bloquant la douille rotative (16) sur le bras de visée (14) dans les différentes positions de rotation.

2. Appareil de visée suivant la revendication 1, **caractérisé en ce qu'**au bras de visée (14), est relié un dispositif de serrage, avec lequel est réalisé un faible coulissement axial de la douille rotative (16) sur le bras de visée (14) pour bloquer une douille de visée et de forage, qui est reçue par un alésage du bras de visée (14) et par une ouverture (18) de la douille rotative (16).

3. Appareil de visée suivant l'une des revendications 1 à 2, **caractérisé en ce que** la douille (16) présente, à son extrémité arrière, une saillie intérieure (46) qui coopère avec une rainure annulaire (26) du bras de visée (14).

4. Appareil de visée suivant la revendication 3, **caractérisé en ce que** la saillie (24) agit en coordination également avec des évidements de blocage (34) du bras de visée (14) qui sont orientés vers la rainure annulaire (26).

5. Appareil de visée suivant l'une des revendications 1 à 4, **caractérisé en ce que** le dispositif de serrage présente un bouton tournant (20) qui, placé à l'extrémité libre de la douille rotative (16), est installé de façon à pouvoir tourner au moyen d'un ergot central (70) dans un évidement (30) du bras de visée, et le bouton tournant (20) et la douille rotative (16) coopèrent, au moyen d'une courbe de guidage et d'un curseur, de telle façon que, lors d'une rotation du bouton tournant (20), la douille rotative (16) soit soumise à une précontrainte dans la direction axiale.

6. Appareil de visée suivant la revendication 5, **caractérisé en ce que** le bouton tournant (20) a une forme de pot et présente sur sa face intérieure au centre, sur le fond, une pièce rapportée (68), à l'extrémité supérieure de laquelle est disposé l'ergot (70) et qui présente, sur sa périphérie, une partie de rainure annulaire (76), qui coopère avec une saillie radiale (48, 50) de la douille rotative (16), la partie de rainure annulaire (76) étant relié à l'extrémité libre de la pièce rapportée (68) par une rainure (78) parallèle à l'axe, permettant l'introduction de la saillie (48, 50) dans la partie de rainure annulaire (76).

7. Appareil de visée suivant la revendication 6, **caractérisé en ce que** la pièce rapportée (68), en tant que pièce séparée, est fixée dans le bouton tournant (20) en forme de pot.

8. Appareil de visée suivant l'une des revendications 5 à 7, **caractérisé en ce qu'**entre le bouton tournant (20) et l'extrémité libre de la douille rotative (16), est disposé un ressort qui crée une précontrainte réciproque entre les pièces.

9. Appareil de visée suivant la revendication 8, **caractérisé en ce qu'**il est prévu un ressort en forme de rondelle.
